# EUROPÄISCHE PATENTSCHRIFT

(11) **EP 1 105 103 B1**
(45) Veröffentlichungstag und Bekanntmachung des Hinweises auf die Patenterteilung: **25.06.2003**
(21) Anmeldenummer: 99941596.1
(22) Anmeldetag: 11.08.1999
(51) Int. Cl.: A61K 9/20

(54) **EXAKT TEILBARE TABLETTE**
EXACTLY DIVISIBLE TABLET
COMPRIME SECABLE AVEC PRECISION

(30) Priorität: 19.08.1998 DE 19837684
(43) Veröffentlichungstag der Anmeldung: 13.06.2001
(73) Patentinhaber: Abbott GmbH & Co. KG, 65205 Wiesbaden (DE)
(72) Erfinder: SPENGLER, Reinhard, D-67133 Maxdorf (DE)
(74) Vertreter: Grünecker, Kinkeldey, Stockmair & Schwanhäusser Anwaltssozietät
(86) Internationale Anmeldenummer: EP9905905
(87) Internationale Veröffentlichungsnummer: WO00010535

(56) Entgegenhaltungen:
- EP-A- 0 207 888
- DE-A- 1 492 237
- DE-B- 1 200 790

## Beschreibung

Die vorliegende Erfindung betrifft eine exakt teilbare Tablette.

Nahezu alle Tabletten mit pharmazeutischen Wirkstoffen können durch die Wahl der Größe und Form und die Einprägung einer oder mehrerer Bruchkerben manuell teilbar gemacht werden. Die bislang bekannten Tabletten sind jedoch entweder nur in einer Richtung teilbar, was bei älteren Patienten häufig zu Problemen führt, oder die Tabletten lassen sich nicht ausreichend präzise in Tablettenhälften mit derselben Wirkstoffmenge teilen. EP-A-0 207 888 offenbart leicht teilbare Tabletten, bei denen eine oder beide Stirnflächen vom abgschrägten Tablettenrand nach innen zur gegenüberliegenden Stirnfläche geneigte schiefe Ebenen haben sowie eine Bruchrille am Scheitelpunkt der sich treffenden schiefen Ebenen aufweisen. Gemäß einem Vorschlag der Europäischen Arzneibuchkommission (Report 96th Meeting) dürfen die Gewichtsanteile der Wirkstoffe in den Tablettenhälften bei einem Wirkstoffgehalt der ganzen Tablette von über 250 mg um maximal 10 % und bei einem Wirkstoffgehalt der ganzen Tablette von unter 250 mg maximal 15 % in der Masse abweichen. Da auch der Wirkstoffgehalt in den einzelnen Tabletten bereits je nach angestrebter Wirkstoffmenge gemäß Deutschem Arzneibuch (10. Auflage) je nach Tablettengröße um 5 bis 10 % vom angestrebten Wert abweichen kann, können bei der Applikation von geteilten Tabletten durch Schwankungen in der applizierten Wirkstoffmenge Schwierigkeiten auftreten.

Es wurde nun eine Tablette gefunden, die sich in zwei Richtungen teilen läßt und die bei der Teilung in zwei Hälften zerbricht, die in ihrer Wirkstoffmenge weitgehend übereinstimmen.

Gegenstand der Erfindung ist eine Tablette mit zwei Stirnflächen und einer Mantelfläche, welche dadurch gekennzeichnet ist, daß die einander gegenüberliegenden Stirnflächen der Tablette
a) insgesamt nicht-eben und zumindest annähernd parallel zueinander angeordnet sind und
b) mit je einer Bruchrille auf der Ober- und Unterseite versehen sind, wobei die Bruchrillen in der Tablettenmitte parallel übereinander liegen.

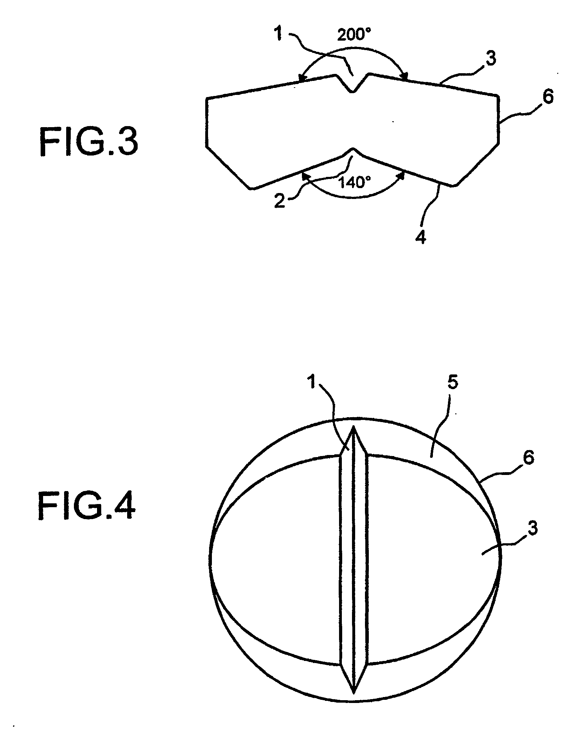

Die einander gegenüberliegenden Stirnflächen der Tablette liegen vorzugsweise parallel oder annähernd parallel zueinander. Die beiden Hälften der Tablette sollen dabei einen Winkel von 130 bis 177° bilden. Bei kleinen Tabletten liegt er vorzugsweise näher an 130°, bei größeren Tabletten kann er bis zu 177° groß sein. Er muß in jedem Fall so groß sein, daß die Tablette bei Druck auf die Ober- bzw. Unterseite gut bricht. Bevorzugt laufen die einander gegenüberliegenden Stirnflächen zueinander parallel. Bei größeren Tabletten kann der Winkel, den die untere Stirnfläche (vgl. Fig. 3) bildet, etwas kleiner (d.h. bis zu 25°, vorzugsweise bis zu 15°) sein, wenn man das Volumen der Tablette vergrößern will, ohne die Brucheigenschaften zu beeinflussen.

Die Bruchrillen haben in der Regel eine Tiefe von 5 bis 10 % der Tablettendicke an der Bruchrille. Die Form der Bruchrillen weist keine Besonderheiten auf.

Es ist zweckmäßig, die Tabletten randseitig auf der Ober- oder Unterseite, vorzugsweise an beiden Seiten, mit einer Phase (= Facette, Schräge) zu versehen, wodurch die Belastbarkeit der Stempel beim Pressen der Tablette vergrößert wird. Hat die Tablette an der Ober- und Unterseite eine Phase, so sind diese um etwa 90° gegeneinander verschoben.

Die Form der Tabletten ist normalerweise rund. Sie kann aber auch oval (beispielsweise oblong) oder vieleckig (beispielsweise rechteckig bzw. quadratisch) sein, wobei die Ecken abgerundet sind. Die Tabletten sollen einen Durchmesser von mindestens 4 mm bis 14 mm besitzen, wenn sie zur oralen Applikation vorgesehen sind.

Die neue Tablette hat folgende Vorteile:
1. Die Tablette kann auf einer Unterlage durch Druck von oben (beispielsweise mit einem Finger) leicht geteilt werden. Es spielt dabei keine Rolle, mit welcher Seite die Tablette auf der Unterlage aufliegt. Letzteres ist besonders wichtig, wenn die Tablette von Personen gebrochen werden muß, die nur schwer sehen können.
2. Der Verlust an Tablettenmasse beim Brechvorgang der Tablette ist minimal.
3. Die Standardabweichung der Bruchstücke hinsichtlich ihrer Masse liegt <3 %.
4. Die Tablette besitzt eine angemessen hohe Bruchfestigkeit, was wichtig für galenische Folgearbeitsschritte ist, bei denen die Tablette eine bestimmte Festigkeit bei gleichzeitig günstiger niedriger Bruchkraft besitzen muß.
5. Dadurch, daß die Tablette an ihrer Oberfläche weitgehend ebene Flächen besitzt, können leicht Gravuren zur Erkennung der Tablette angebracht werden, ohne daß die Tablette beschädigt wird, auch bei hoher Tablettiergeschwindigkeit nicht. Bei gewölbten Oberflächen gelingt das nur mit einem viel größeren Aufwand.
6. Beim Tablettierprozeß kann die neue Tablette aufgrund ihrer halbmondförmigen Facette besonders leicht aus der Preßform herausgleiten, so daß auch bei hoher Preßgeschwindigkeit eine Beschädigung der Tablette beim Abstreifvorgang nicht auftritt.

Die Figuren zeigen spezielle Ausführungsformen der Erfindung.

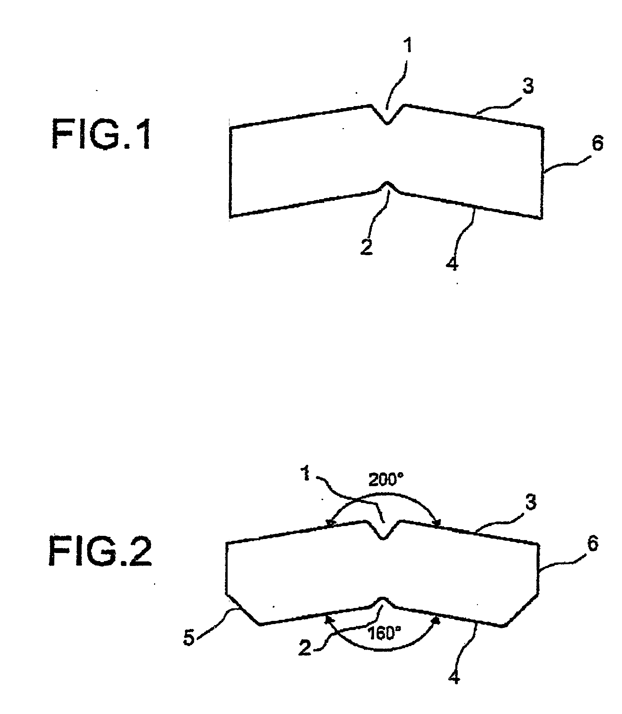

- Fig. 1: zeigt einen Querschnitt durch eine Tablette. 1 und 2 sind die Bruchrillen, 3 ist die obere und 4 die untere Stirnfläche und 6 die Mantelfläche der Tablette.
- Fig. 2: zeigt einen Querschnitt durch eine Tablette gemäß Fig. 1, an der zusätzlich eine Phase 5 angebracht ist.
- Fig. 3: entspricht der Fig. 2, jedoch ist die untere Stirnfläche stärker gewinkelt.
- Fig. 4: ist eine Sicht auf die Tablette gemäß Fig. 2. 1 ist eine Bruchrille, 3 ist die obere Stirnfläche mit der Phase 5.

## Patentansprüche

1. Tablette mit zwei Stirnflächen und einer Mantelfläche, **dadurch gekennzeichnet, daß** die einander gegenüberliegenden Stirnflächen der Tablette
a) insgesamt nicht-eben und zumindest annähernd, parallel zueinander angeordnet sind und
b) mit je einer Bruchrille auf der Ober- und Unterseite versehen sind, wobei die Bruchrillen in der Tablettenmitte parallel übereinander liegen sowie **dadurch gekennzeichnet, daß** die Tablette randseitig auf der Ober- und/oder Unterseite mit je zwei halbmondförmigen Phasen versehen ist.

2. Tablette gemäß Ansprach 1, **dadurch gekennzeichnet, daß** die durch die Bruchrille geteilten Hälften der Tablette einen Winkel von 130 bis 177° bilden.

## Claims

1. Tablet with two end faces and a circumferential face, **characterized in that** the mutually opposite end faces of the tablet
a) are on the whole non-planar and are arranged at least approximately parallel to one another, and
b) are provided with a score on each of the top and bottom sides, the scores lying in the centre of the tablet parallel to and above one another,
and **characterized in that** the tablet is provided at the edges, on the top and/or bottom side, with two crescent-shaped facets in each case.

2. Tablet according to Claim 1, **characterized in that** the halves of the tablet divided by the score form an angle of 130 to 177°.

## Revendications

1. Comprimé ayant deux surfaces frontales et une surface d'enveloppe, **caractérisé en ce que** les surfaces frontales du comprimé qui sont opposées l'une à l'autre
a) sont agencées globalement de manière non plane et au moins sensiblement parallèlement l'une à l'autre et
b) sont munies chacune d'une rainure de cassure sur le côté supérieur et inférieur, les rainures de cassure étant situées parallèlement l'une au-dessus de l'autre au centre du comprimé, et **caractérisé en ce que** le comprimé est muni en bordure sur le côté supérieur et/ou inférieur de deux phases en demi-lune.

2. Comprimé selon la revendication 1, **caractérisé en ce que** les moitiés du comprimé séparées par la rainure de cassure forment un angle de 130 à 177°.
